(19) **Europäisches Patentamt**

**European Patent Office**

**Office européen des brevets**

(11) **EP 4 656 207 A1**

(12) **EUROPEAN PATENT APPLICATION**

(43) Date of publication:
03.12.2025 Bulletin 2025/49

(21) Application number: 24382581.7

(22) Date of filing: **30.05.2024**

(51) International Patent Classification (IPC):
*A61K 47/00* (2006.01)   *A61K 49/00* (2006.01)
*C07K 19/00* (2006.01)   *C07K 17/00* (2006.01)
*A61K 38/00* (2006.01)   *C12N 15/86* (2006.01)

(52) Cooperative Patent Classification (CPC):
**C12N 15/86; C07K 14/78; C12N 15/87;**
C07K 2319/10; C12N 2740/16043

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Fundació Hospital Universitari Vall Hebron - Institut de Recerca**
**08035 Barcelona (ES)**
• **Universitat de Lleida**
**25003 Lérida (ES)**
• **Universitat Autònoma de Barcelona**
**08193 Cerdanyola del Vallès (Barcelona) (ES)**

(72) Inventors:
• **BARQUINERO MAÑEZ, Jordi**
**E-08035 BARCELONA (ES)**

• **VERDAGUER AUTONELL, Juan**
**E-25003 Lleida (ES)**
• **CORRAL PUJOL, Marta**
**E-25003 Lleida (ES)**
• **PIÑERA MORENO, Rocío**
**E- 30530 Cieza (Murcia) (ES)**
• **TORT BARDOLET, Lluis**
**E-08012 Barcelona (ES)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **IN VITRO METHOD FOR TRANSDUCING A TARGET CELL**

(57)    The invention relates to a method for the transduction of target cells, as well as to products for improving the transduction yield.

**EP 4 656 207 A1**

**Description**

## TECHNICAL FIELD OF THE INVENTION

The present invention relates to a method for the transduction of target cells, as well as to products for improving the transduction yield.

## BACKGROUND OF THE INVENTION

After more than three decades of research in gene therapy (GT), the discipline has reached a point in which many strategies, vectors and medicinal products have been generated, several of them have demonstrated clinical efficacy, and some have already obtained market authorization by the FDA and/or the EMA. The number of commercial GT products in the market increases every year, both for *in vivo* (vectors) and for *ex vivo* applications (gene-modified cells). Indeed, for *ex vivo* GT, the field is now expanding very fast due to the success of CAR-T cells and the emerging CAR-NK cells in cancer immunotherapy. *Ex vivo* GT is based on the use of vectors (usually viral) to genetically modify cells isolated from patients that are subsequently reintroduced for therapeutic purposes. In addition to the mentioned immunotherapies, another field of applications for *ex vivo* GT is hematopoietic stem cell gene therapy, mainly applicable to hereditary diseases of the hematopoietic system, which include sickle cell disease, β-thalassemia, primary immunodeficiencies or Fanconi anemia, among others.

Despite these advances, transduction of some of these cell types with current viral vectors is still relatively inefficient, and improving it safely without compromising cell functionality is an unmet clinical need. Potential solutions to increase transduction rates include the use of physical procedures such as spinoculation, or to use higher Multiplicity Of Infection (MOI, the amount or proportion of vector particles used per target cell), but this has the disadvantage of making the procedure substantially more expensive, as the cost of producing these vectors, which must be produced in Good Manufacturing Practice (GMP) conditions as they are intended to be used in humans. These manufacturing represents approximately a quarter of the cost of the final drug, which have market prices up to 2 - 3 million euros per patient/-procedure. Thus, the use of chemical compounds that improve the transduction efficiency (transduction enhancers or TEs) may allow to substantially reduce the MOI and thus the amount of vector that is required to achieve therapeutic levels of gene transfer, thus reducing the costs of manufaturing the gene therapy medicinal products (GTMP), and this can also improve its safety. In addition, there is a second problem, that some cell types such as NK cells are intrinsically resistant to transduction with lentiviral vectors, especially those based on the VSV-G pseudotype (the only one approved for clinical use to date), which makes it necessary to find better strategies or TEs to improve and optimize the production of CAR-NK cells, a highly promising form of immunotherapy for cancer. The use of clinical grade transduction enhancers (TE) may allow a significant cut (by several times) in the amounts of vectors required for an efficient transduction, thus reducing the costs of the *ex vivo* GT medicinal products.

## SUMMARY OF THE INVENTION

In 2017, the group of Dr. J. Verdaguer (UdL) serendipitously discovered a family of peptides derived from intermediate filaments (IF) that showed strong immunostimulatory activity in the micromolar range (EP3756680A1). In the present invention, the inventors have discovered that said peptides derived from intermediate filaments are able to increase the yield of cellular transduction by viral vectors.

Therefore, a first aspect of the present invention relates to an *in vitro* method for transducing a target cell comprising:

(a) providing a peptide comprising, in order going from the N-terminal end to the C-terminal end:

(i) a first region consisting of an amino acid sequence of "n" amino acids, wherein "n" is 0 to 41 amino acids;

(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I] and

(iii) a third region consisting of an amino acid sequence of "m" amino acids, wherein "m" is 0 to 41 amino acids,

wherein said peptide has a minimum length of 9 amino acids and a maximum length of 50 amino acids,

(b) contacting the target cell with a viral vector and the peptide according to step (a), and

(c) incubating the target cell in a culture medium.

A further aspect of the present invention relates to a conjugate for enhancing transduction efficiency comprising:

(a) a peptide comprising, in order going from the N-terminal end to the C-terminal end:

(i) a first region consisting of an amino acid sequence of "n" amino acids, wherein "n" is 0 to 41 amino acids;

(ii) a second region consisting of the amino acid sequence [A/I/L/S/T/V]-[K/R]-[L/I/M/S/T/-V/A]-[G/R/A/H/K/S/F]-[L]-[D/E]-[IiNN IM/K/QI A/L/G/C]-[E]-[I] and

(iii) a third region consisting of an amino acid sequence of "m" amino acids, wherein "m" is 0 to 41 amino acids,

wherein said peptide has a minimum length of 9 amino acids and a maximum length of 50 amino acids, and

(b) an amphiphilic polymer,

wherein the amphiphilic polymer is conjugated to the peptide.

Another aspect of the present invention relates to the use of the conjugate according to the invention for enhancing the transduction of a target cell.

One more aspect of the present invention relates to a method for obtaining a conjugate according to the invention comprising:

(a) providing a peptide comprising, in order going from the N-terminal end to the C-terminal end:

(i) a first region consisting of an amino acid sequence of "n" amino acids, wherein "n" is 0 to 41 amino acids;

(ii) a second region consisting of the amino acid sequence [A/I/LVS/T/V]-[K/R]-[L/I/M/S/T/-V/A]-[G/R/A/H/K/S/F]-[L]-[D/E]-[IiNN IM/K/QI A/L/G/C]-[E]-[I] and

(iii) a third region consisting of an amino acid sequence of "m" amino acids, wherein "m" is 0 to 41 amino acids, wherein said peptide has a minimum length of 9 amino acids and a maximum length of 50 amino acids, and

an amphiphilic polymer, and

(b) conjugating the peptide with an activated form of the amphiphilic polymer or a derivative thereof, wherein said activated form of the amphiphilic polymer contains a reactive group which is capable of reacting with at least one group in the peptide and wherein the contact is carried out under conditions adequate for the formation of a bond between the reactive group in the amphiphilic polymer and the group in the peptide.

A further aspect of the present invention relates to a conjugate for enhancing transduction efficiency obtained by the method according to the previous aspect.

One more aspect relates to a kit comprising a conjugate according to the invention.

A final aspect of the present invention relates to the use of the kit according to the invention for the transduction of a target cell.

## BRIEF DESCRIPTION OF THE FIGURES

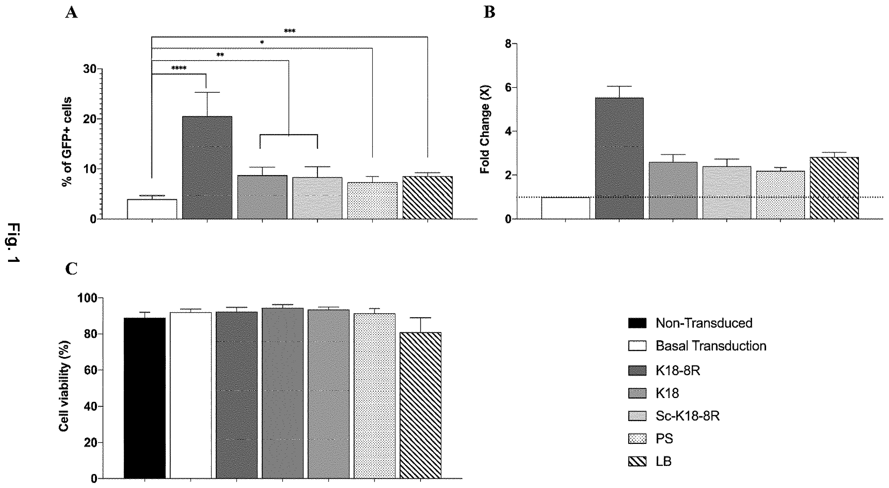

**Figure 1.** Results of the transduction experiments in NIH/3T3 cells, comparing different TEs, at MOI = 1. A) Chart represents the transduction efficiencies estimated by the percentages of GFP$^+$ cells in the transduced samples measured by flow cytometry 72 h after vector exposure. Chart B contains the same information, but normalized by Fold Change (X) relative to the Basal Transduction (value = 1). Chart C represent the percentages of living cells also analyzed by flow cytometry 72 h after transduction. Non-Transduced: cells not exposed to lentiviral vectors; Basal Transduction: transduction made in the absence of TE; K18-8R, K18 and Sc-K18-R are the micellar peptides described in the Methodology section (all used at 1 $\mu$M), LB: LentiBoost$^{TM}$ (1 mg/mL); PS: Protamine sulfate. $^*$ p<0.05, $^{**}$ p<0.01, $^{***}$ p<0.001, $^{****}$ p<0.0001. N = 5.

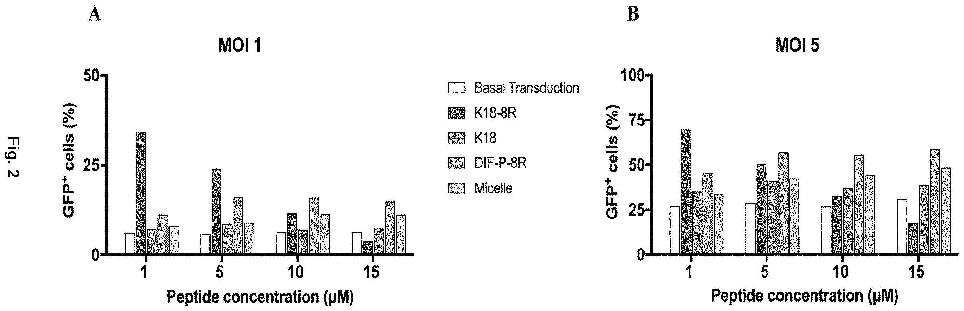

**Figure 2.** Results of transduction experiments in HEK-293T cells, comparing different peptides, at different concentrations, at MOI = 1 (A) and 5 (B). Basal Transduction: transduction made in the absence of TE; K18-8R, K18 and DIF-P-8R (all at 1 $\mu$M) are the micellar peptides. N = 2.

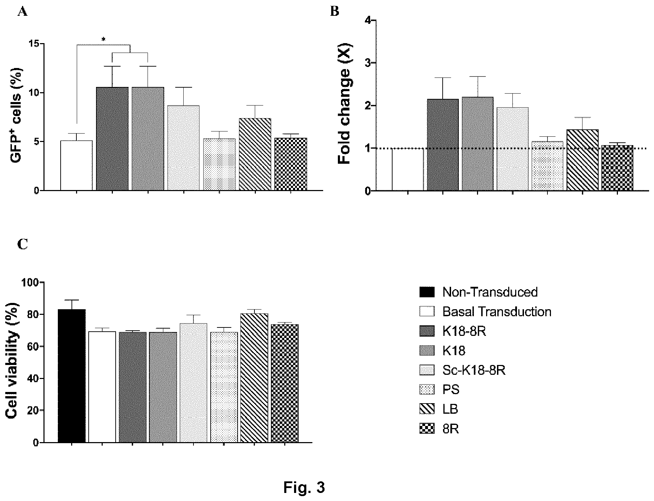

**Figure 3.** Results of transduction experiments in HEK-293T cells, comparing different TE, at MOI = 1. A) Chart represents the transduction efficiencies estimated by the percentages of GFP$^+$ cells in the transduced samples measured by flow cytometry 72 h after vector exposure. Chart B contains the same information, but relativized by Fold Change relative to the Basal Transduction (value = 1). Chart C represent the percentages of living cells also analyzed by flow cytometry 72 h after transduction. Non-Transduced: cells not exposed to lentiviral vectors; Basal Transduction: transduction made in the absence of TE; K18-8R, K18, Sc-K18-8R and 8R (all at 1 $\mu$M) are the micellar peptides, LB: LentiBoost™ (1 mg/mL); PS: Protamine sulfate. * p<0.05. N = 5.

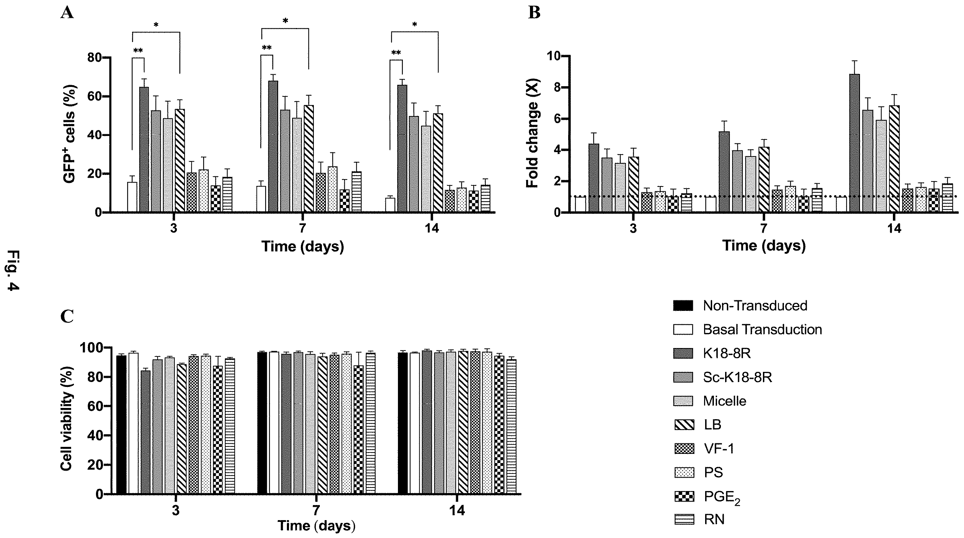

**Figure 4.** Results of a representative transduction experiment in CD34$^+$ hematopoietic progenitors, comparing the different TEs, analyzed by flow cytometry at different times (3, 7 and 14 days) after transduction. MOI = 1. A) Chart represents the transduction efficiencies estimated by the percentages of GFP$^+$ cells in the transduced samples. Chart B contains the same information, but normalized by Fold Change relative to the Basal Transduction (value = 1). Chart C represent the percentages of living cells also analyzed by flow cytometry 72 h after transduction. Non-Transduced: cells not exposed to lentiviral vectors; Basal Transduction: transduction in the absence of TE; K18-8R and Sc-K18-R (all at 3 $\mu$M) are the micellar peptides, LB: LentiBoost™ (5 mg/mL); PGE$_2$: Prostaglandin E$_2$; PS: Protamine sulfate; RN: Retronectin™ ; VF-1: Vectofusin-1™. * p<0.05, ** p<0.01. N = 3.

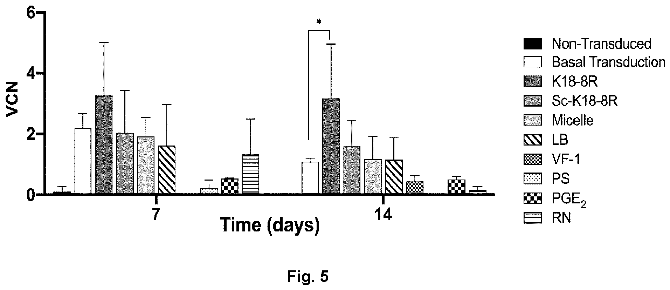

**Figure 5.** Results of transduction experiments in CD34$^+$ cells, comparing the different TEs, analyzed by qPCR. MOI = 1. Chart represents the Vector Copy Number (VCN) calculated in the transduced samples. Non-Transduced: cells not exposed to lentiviral vectors; Basal Transduction: transduction in the absence of TE; K18-8R and Sc-K18-R are the micellar peptides (at 3 $\mu$M), LB: LentiBoost™ (5 mg/mL); PGE$_2$: Prostaglandin E$_2$; PS: Protamine sulfate; RN: Retronectin™ ; VF-1: Vectofusin-1™. * p<0.05. N = 3.

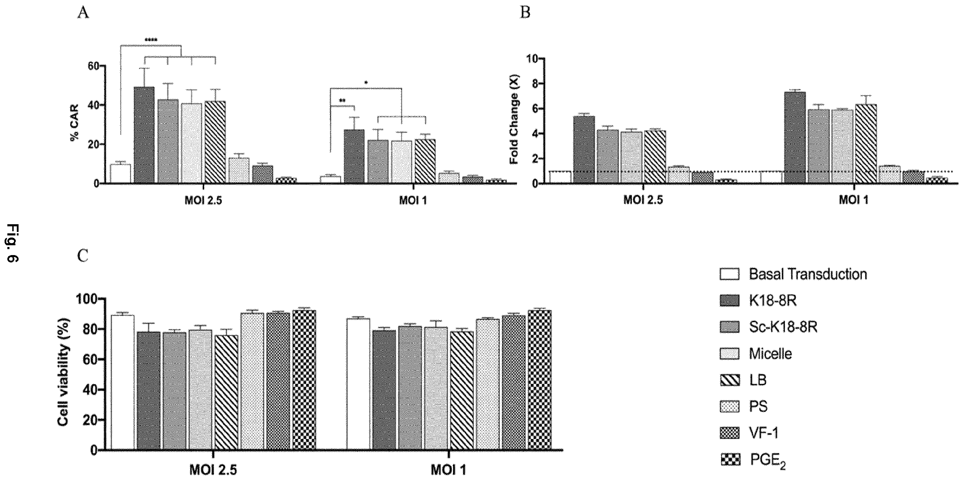

**Figure 6.** Results of T-cell transduction experiments, comparing the different TEs. MOIs = 1 and 2.5. A) Chart represents the transduction efficiencies estimated by the percentages of GFP$^+$ cells in the transduced samples. Chart B contains the same information, but normalized by Fold Change relative to the Basal Transduction (value = 1). Chart C represent the percentages of living cells also analyzed by flow cytometry 72 h after transduction. Non-Transduced: cells not exposed to lentiviral vectors; Basal Transduction: transduction in the absence of TE; K18-8R and Sc-K18-R are the micellar peptides (at 3 $\mu$M), LB: LentiBoost™ (5 mg/mL); PGE$_2$: Prostaglandin E$_2$; PS: Protamine sulfate; VF-1: Vectofusin-1™. * p<0.05, ** p<0.01, **** p<0.0001. N = 3.

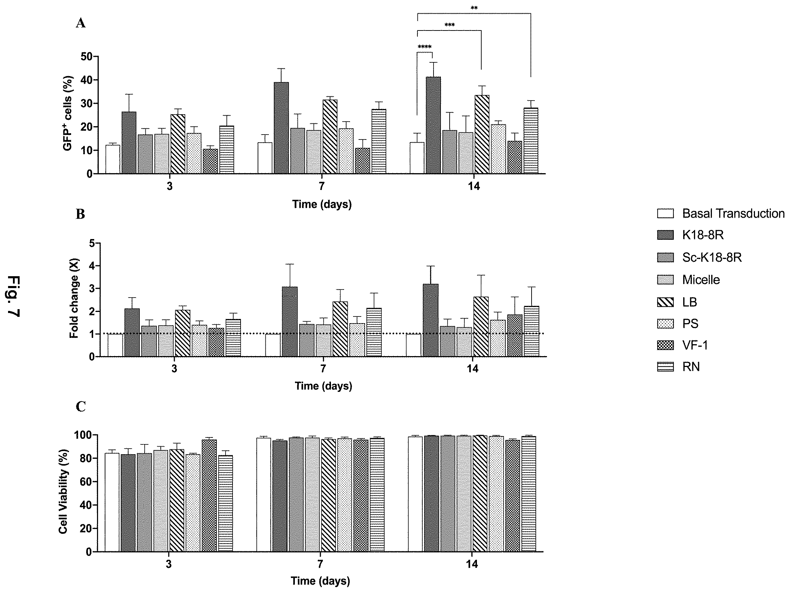

**Figure 7.** Results of transduction experiments in primary human NK cells, comparing our peptide compounds with a panel of commercial TE, using a MOI = 5. A) Chart represents the transduction efficiencies estimated by the percentages of GFP$^+$ cells in the transduced samples at days 3, 7 and 14 after transduction. B) Results normalized by Fold Change relative to the Basal Transduction (value = 1). C) Percentages of living cells analyzed by flow cytometry at the three timepoints. Non-Transduced: cells not exposed to lentiviral vectors; Basal Transduction: transduction in the absence of TE; K18-8R and Sc-K18-R are the micellar peptides (1.5 $\mu$M), LB: LentiBoost™ (2.5 mg/mL); PS: Protamine sulfate; RN: Retronectin™ ; VF-1: Vectofusin-1™. ** p<0.01, *** p<0.001, **** p<0.0001. N = 3.

## DETAILED DESCRIPTION OF THE INVENTION

In the present invention, the inventors have discovered that adding peptides derived from intermediate filaments into a reaction of transduction of a target cell leads to the surprising effect of promoting the transduction of viral vectors in said cell.

*Method of transduction*

Hence, a first aspect of the present invention relates to an *in vitro* method for transducing a target cell, from here onwards the method of the invention, comprising:

(a) providing a peptide comprising, in order going from the N-terminal end to the C-terminal end:

(i) a first region consisting of an amino acid sequence of "n" amino acids, wherein "n" is 0 to 41 amino acids;

(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I] and

(iii) a third region consisting of an amino acid sequence of "m" amino acids, wherein "m" is 0 to 41 amino acids,

wherein said peptide has a minimum length of 9 amino acids and a maximum length of 50 amino acids,

(b) contacting the target cell with a viral vector and the peptide according to step (a), and

(c) incubating the target cell in a culture medium.

The term "transduction", as used herein, refers to the process whereby a foreign nucleic acid is introduced into a cell, so called "target cell" via a viral vector. Suitable conditions for transducing a cell with a recombinant viral vector are known by the skilled person. Transduction can result in a "stable transduction", wherein the nucleic acid transferred is stably integrated into the genome. On another hand, transduction can result in "transient transduction" or "transiently transduced" cells, wherein the foreign DNA fails to integrate into the genome of the transduced cell. The foreign DNA can persists in the nucleus of the transduced cell for several days (up to several years). During this time the foreign DNA is subjected to the regulatory controls that govern the expression of endogenous genes in the chromosomes. The term "transient transductant" refers to cells that have taken up foreign DNA but have failed to integrate this DNA.

The term "in vitro" as used herein refers to the method being carried out outside a living organism, wherein the components of organisms are isolated from their usual biological context in order, in an artificial environment. In a particular embodiment of the method of the invention, the method is an ex vivo method.

The term "*ex vivo*" refers to being outside of a living organism. In the context of the present invention, the ex-vivo method is performed in cells which are outside a living organism, for example which are grown in a test tube, and which may be subsequently returned to the organism.

As used herein, the term "target cells" refers to cells that are the target of the transduction. Examples of target cells are human cells or murine cells. In a preferred embodiment of the method of the invention, the target cell is a human cell or a murine cell, in particular a human cell. In a preferred embodiment of the method of the invention, the target cell is a primary cell. In another preferred embodiment of the method of the invention, the target cell is a cell selected from the group consisting of a lymphocyte, a tumor cell, a lymphoid lineage cell, a neuronal cell, an epithelial cell, a keratinocyte, an endothelial cell, a T cell, a haematopoietic cell, and a stem cell.

The term "haematopoietic cell" as used herein refers to a cell of the haematopoietic system that is capable of differentiating into differentiated cells such as a monocyte and a T-lymphocyte. The term "haematopoietic cell" thus encompasses a haematopoietic stem cell, as well as a progenitor cell differentiated from a haematopoietic stem cell, (haematopoietic progenitor cell), wherein said progenitor cell is capable of differentiating into several different cells just as monocytes, macrophages, and dendritic cells. A haematopoietic cell in accordance with the present invention may relate to a haematopoietic stem cell, a haematopoietic progenitor cell or combinations thereof.

In a further preferred embodiment of the method of the invention, the target cell is a haematopoietic cell, wherein the haematopoietic cell is a haematopoietic stem cell, a haematopoietic progenitor cell, a CD34+ cell, a monocyte, a macrophage, a tissue resident macrophage, a microglial cell, or a dendritic cell. In yet another preferred embodiment of the method of the invention, the target cell is a murine cell, wherein the murine cell is NIH/3T3, RAW64 or A-20. In a further preferred embodiment of the method of the invention, the target cell is a human cell, wherein the human cell is HEK-293T. In one more embodiment of the method of the invention, the target cell is a lymphocyte, wherein the lymphocyte is a T cell, a B cell or a natural killer (NK) cell.

The term "peptide", as used herein, refers to a sequence of amino acids, analogues or mimetics having substantially similar or identical functionality. The term "peptide" also includes analogues having synthetic and natural amino acids joined together by peptide bonds. A peptide, as used herein and in the claims, is also intended to include analogues, derivatives, salts, retro-inverso isomers, mimics, mimetics, or peptidomimetics thereof. For example, a peptidic structure of a modulator of the invention may be further modified to increase its stability, bioavailability, solubility, etc. "Analog", "derivative" and "mimetic" include molecules which mimic the chemical structure of a peptidic structure and retain the functional properties of the peptidic structure. Approaches to design peptide analogues, derivatives and mimetics are known in the art. For example, see Farmer, P. S. in Dmg Design (E. J. Ariens, ed.) Academic Press, New York, 1980, vol. 10, pp. 119-143; Ball, J. B. and Alewood, P. F. (1990) J. Mol. Recognition 3:55. Morgan, B. A. and Gainor, J. A. (1989) Ann. Rep. Med. Chem. 24:243; and Freidinger, R. M. (1989) Trends Pharmacol. Sci. 10:270. See also Sawyer, T. K. (1995) Peptidomimetic Design and Chemical Approaches to Peptide Metabolism in Taylor, M. D. and Amidon, G. L. (eds.) Peptide-Based Drug Design: Controlling Transport and Metabolism, Chapter 17; Smith, A. B. 3rd, et al. (1995) J. Am. Chem. Soc. 117:11113-11123; Smith, A. B. 3rd, et al. (1994) J. Am. Chem. Soc. 116:9947-9962; and Hirschman, R., et al. (1993) J. Am. Chem. Soc. 115:12550-12568. A "derivative" (e.g., a peptide or amino acid) includes forms in which one or more reaction groups on the compound have been derivatised with a substituent group. Examples of peptide derivatives include peptides in which an amino acid side chain, the peptide backbone, or the amino- or carboxy-terminus has been derivatised (e.g., peptidic compounds with methylated amide linkages). An "analogue" of a compound X includes compounds which retain chemical structures necessary for functional activity, yet which also contains certain chemical structures that differ. An example of an analogue of a naturally-occurring peptide is a peptide which includes one or more non-naturally-occurring amino acids. A "mimetic" of a compound includes compounds in which chemical structures of the compound necessary for functional activity have been replaced with other chemical structures that mimic the conformation of the compound. Examples of peptidomimetics include peptidic compounds in which the peptide backbone is substituted by one or more benzodiazepine molecules (see e.g., James, G. L. et al. (1993) Science 260:1937-1942).

The term "amino acid" refers to naturally occurring and synthetic amino acids, as well as amino acid analogues and mimetics that function in a manner similar to the naturally occurring amino acids. Amino acids may be referred to herein by either their commonly known three letter symbols or by the one-letter symbols recommended by the IUPAC-IUB Biochemical Nomenclature Commission. The term amino acid includes naturally occurring amino acids (Ala, Arg, Asn, Asp, Cys, Gln, Glu, Gly, His, Ile, Leu, Lys, Met, Phe, Pro, Ser, Thr, Trp, Tyr, Val), uncommon natural amino acids, and non-natural (synthetic) amino acids. The amino acids are preferably in the L configuration, but also D configuration, or mixtures of amino acids in the D and L configurations. The term "natural amino acids" comprises aliphatic amino acids (glycine, alanine, valine, leucine and isoleucine), hydroxylated amino acids (serine and threonine), sulfured amino acids (cysteine and methionine), dicarboxylic amino acids and their amides (aspartic acid, asparagine, glutamic acid and glutamine), amino acids having two basic groups (lysine, arginine and histidine), aromatic amino acids (phenylalanine, tyrosine and tryptophan) and cyclic amino acids (proline). As used herein, the term "non-natural amino acid" refers to a carboxylic acid, or a derivative thereof, substituted at position $\alpha$ with an amine group and being structurally related to a natural amino acid. Illustrative non-limiting examples of modified or uncommon amino acids include: 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allohydroxylysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

Amino acid positions found depicted in brackets indicate that the amino acids found within the brackets can be found as alternatives in the given position. For example:

- [A/I/L/S/T/V] indicates that the amino acid at said position can be Ala, Ile, Leu, Ser, Thr, or Val;
- [K/R] indicates that the amino acid at said position can be Lys or Arg;
- [L/I/M/S/T/V/A] indicates that the amino acid at said position can be Leu, Ile, Met, Ser, Thr, Val, or Ala;
- [G/R/A/H/K/S/F] indicates that the amino acid at said position can be Gly, Arg, Ala, His, Lys, Ser, and Phe;
- [L] indicates that the amino acid at said position can only be Leu;
- [D/E] indicates that the amino acid at said position can be Asp or Glu;
- [I/N/V/M/K/Q/A/L/G/C] indicates that the amino acid at said position can be Ile, Asn, Val, Met, Lys, Gln, Ala, Leu, Gly or Cys;
- [E] indicates that the amino acid at said position can only be Glu;
- [I] indicates that the amino acid at said position can only be Ile.

The peptide of the method of the invention is characterized by comprising three regions. The second region of the peptide of the method of the invention consists of an amino acid sequence of 9 amino acids. The amino acid in position 1 of the second region of the peptides of the invention is Ala, Ile, Leu, Ser, Thr, or Val, most preferably Ile. The amino acid in position 2 of the second region of the peptides of the invention is Lys or Arg; preferably Lys. The amino acid in position 3 of the second region of the peptides of the invention is Leu, Ile, Met, Ser, Thr, Val, or Ala; most preferably Val. The amino acid in position 4 of the second region of the peptides of the invention Gly, Arg, Ala, His, Lys, Ser, and Phe; most preferably Lys. The amino acid in position 5 of the second region of the peptides of the invention is Leu. The amino acid in position 6 of the second region of the peptides of the invention is Asp or Glu, most preferably Glu. The amino acid in position 7 of the second region of the peptides of the invention is Ile, Asn, Val, Met, Lys, Gln, Ala, Leu, Gly or Cys; most preferably Ala. The amino acid in position 8 of the second region of the peptides of the invention is Glu. The amino acid in position 9 of the second region of the peptides of the invention is Ile.

In a particular embodiment, the second region of the peptide of the method of the invention comprises or consists of the sequence according to SEQ ID NO: 1.

SEQ ID NO: 1
IKVKLEAEI

In a particular embodiment, the peptide of the method of the invention has a minimum length of 9 amino acids and a maximum length of 50 amino acids. In another particular embodiment, the peptide of the method of the invention has a minimum length of 12 amino acids and a maximum length of 50 amino acids. In a particular embodiment, the peptide of the method of the invention has a minimum length of 9 amino acids and a maximum length of 20 amino acids. In another particular embodiment, the peptide of the method of the invention has a minimum length of 12 amino acids and a maximum length of 20 amino acids. In another particular embodiment the peptide of the method of the invention has a length of 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids.

The first region of the peptide of the method of the invention consists of an amino acid sequence of "n" amino acids, wherein "n" is 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 amino acids. The third region of the peptide of the method of the invention consists of an amino acid sequence of "m" amino acids, wherein "m" is 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 amino acids. In a particular embodiment, the peptide of the method of the invention has a length of 20 amino acids.

In another preferred embodiment of the peptide of the method of the invention, the first region comprises three amino acids. In another preferred embodiment of the peptide of the method of the invention, the first region comprises the sequence according to Leu Leu Asn (LLN).

The third region of the peptide of the method of the invention is characterized by comprising between 0 and 41 amino acids. In a preferred embodiment of the method of the invention the third region of the peptide comprises 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41 amino acid(s), more preferably 0, 1, 2, 3, 4, 5, 6, 7, 8, 9, 10 amino acids.

In a preferred embodiment of the peptide of the method of the invention, the third region comprises eight amino acids. In another preferred embodiment of the peptide of the method of the invention, the third region comprises or consists of the amino acid sequence according to SEQ ID NO: 2.

SEQ ID NO: 2

*ATYRRLLE*

In a further preferred embodiment of the method of the invention, the peptide comprises or consist of the sequence according to SEQ ID NO: 3.

SEQ ID NO: 3

*LLNIKVKLEAEIATYRRLLE*

In another preferred embodiment of the method of the invention, the peptide comprises in the first region and/or the third region a cell penetrating peptide.

The term "cell penetrating peptide" or "CPPs" as used herein refers to short peptides that facilitate cellular intake/uptake of various molecular entities associated with the peptides either through chemical linkage via covalent bonds or through non-covalent interactions. The associated molecular entities range from nanosize particles to small chemical molecules and large fragments of DNA. The function of the CPPs is to deliver the associated entities into cells, a process that commonly occurs through endocytosis. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only apolar residues, with low net charge or have hydrophobic amino acid groups that are crucial for cellular uptake. In a particular embodiment, the cell penetrating peptide is a tumour-penetrating peptide that comprises the 9-amino acid cyclic peptide iRGD (CRGDKGPDC, SEQ ID NO: 4). In another particular embodiment, the cell penetrating peptide comprises a polar amino acid sequence. In a preferred embodiment, the cell penetrating peptide comprises three or more (i.e. 3, 4, 5, 6, 7, 8 or more) sequential Lysine amino acids (KKK), three or more (i.e. 3, 4, 5, 6, 7, 8, or more) sequential Arginine amino acids (RRR) or three or more (i.e. 3, 4, 5, 6, 7, 8, or more) sequential Histidine amino acids (HHH). In a more preferred embodiment, the cell penetrating peptide comprises eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

In another preferred embodiment of the method of the invention, the peptide of the method of the invention comprises or consists of the sequence according to SEQ ID NO: 8 or SEQ ID NO: 9.

SEQ ID NO: 8

*LLNIKVKLEAEIRRRRRRRR*

In another preferred embodiment, the peptide of the method of the invention is conjugated to an amphiphilic polymer.

The term "amphiphilic polymer" as used herein refers to a polymer that has portions that are non-polar and portions which are polar. The two portions may be evenly distributed in regularly alternating sections along the length of the polymer or in a random arrangement of polar and non-polar sections. In a preferred embodiment, the amphiphilic polymer of the method of the invention is selected from a group consisting of: poloxamer or a carboxylate derivative thereof, polyvinyl caprolactam-polyvinyl acetate-polyethylene glycol graft co-polymer, D-α-Tocopherol polyethylene glycol 1000 succinate, poloxamines, poly(ethylene glycol)-block-poly(ε-caprolactone), and Poly(lactide-co-glycolide)-block-poly(ethylene glycol).

The term "poloxamer" as used herein refers to nonionic triblock copolymers composed of a central hydrophobic chain of polyoxypropylene (polypropylene oxide)) flanked by two hydrophilic chains of polyoxyethylene (poly(ethylene oxide)). Because the lengths of the polymer blocks can be customized, many different poloxamers exist that have slightly different properties. For the generic term poloxamer, these copolymers are commonly named with the letter P (for poloxamer) followed by three digits: the first two digits multiplied by 100 give the approximate molecular mass of the polyoxypropylene core, and the last digit multiplied by 10 gives the percentage polyoxyethylene content (e.g. P407 = poloxamer with a polyoxypropylene molecular mass of 4000 g/mol and a 70% polyoxyethylene content). For the Pluronic and Synperonic tradenames, coding of these copolymers starts with a letter to define its physical form at room temperature (RT) (L = liquid, P = paste, F = flake (solid)) followed by two or three digits, The first digit (two digits in a three-digit number) in the numerical designation, multiplied by 300, indicates the approximate molecular weight of the hydrophobe; and the last digit x 10 gives the percentage polyoxyethylene content (e.g., L61 indicates a polyoxypropylene molecular mass of 1800 g/mol and a 10% polyoxyethylene content). In the example given, poloxamer 181 (P181) = Pluronic L61 and Synperonic PE/L 61.

In a preferred embodiment of the method of the invention, the amphiphilic polymer is a poloxamer or a carboxylated derivative thereof, preferably the poloxamer F-127.

The term "carboxylated derivative" as used herein refers to a polymer which comprises at least one carboxyl group (COOH), preferably being a polycarboxyl polymer, i.e., comprising several carboxyl groups.

The term "conjugate" as used herein refers to means the chemically derivatized product of attaching the peptide of the method of the invention to the amphiphilic polymer.

Techniques for the conjugation of peptides and polymers are well known in the art (view for example Pasut, G., Polymers 2014, 6(1), 160-178; Singh,A. & Kumari, K. & Paban, P. Physical Sciences Reviews, 2023, 1-18). As way of example, the most common method for conjugating a polymer to a peptide is to use its carboxylate derivate version whereby the carboxyl group binds to the peptides through their amino groups, forming an amide bond.

In a preferred embodiment of the method of the invention the peptide is conjugated to an amphiphilic polymer via a covalent bond, preferably an amine bond.

The conjugation of the peptide to the amphiphilic polymer can result in a conjugate with different molar ratio of peptide to polymer. In a preferred embodiment of the method of the invention, the molar ratio of peptide to amphiphilic polymers is of between 1:1 to 1:8, preferably between 1:2 to 1:6, more preferably of 1:4, more preferably the ratio is of 1:1, 1:2, 1:3, 1:4, 1:5, 1:6, 1:7, 1:8.

In a preferred embodiment of the method of the invention, the peptide conjugated to the amphiphilic polymer forms particles (micelles). The term "micelle" used herein refers to aggregates of peptides conjugated to the amphiphilic polymers according to the invention, present in an aqueous solution, wherein the hydrophobic domains of the conjugate aggregates are oriented toward the interior of the micelle and the hydrophilic domains are in contact with the aqueous solution. Micelle structures include, but are not limited to, spherical, laminar, cylindrical, ellipsoidal, vesicular and lamellar.

In a preferred embodiment of the method of the invention, the particles have a diameter of about 20 nm to about 30 nm, preferably about 21 nm, about 22 nm, about 23 nm, about 24 nm, about 25 nm, about 26 nm, about 27 nm, about 28 nm, about 29 nm, about 30 nm. Methods to determine the size of a micelle are well known in the art. Examples are, without limitation, Dynamic Light Scattering (DLS), transmission electron microscopy (TEM), scanning electron microscopy (SEM). In a preferred embodiment of the method of the invention, the diameter of the micelles is measured by DLS as exemplified by Takahashi et al. (Doi: 10.1002/ppsc.200700015).

Step a) of the method of the invention requires the provision of a peptide previously described. In a preferred embodiment of the method of the invention, the peptide is used at a concentration of about 0.5 μM to about 5.0 μM, preferably of 1.0 μM to about 3.0 μM, more preferably about 1.5 μM. In another preferred embodiment of the method of the invention the peptide is used at a concentration of about 0.5 μM, about 0.6 μM, about 0.7 μM, about 0.8 μM, about 0.9 μM, about 1 μM, about 1.1 μM, about 1.2 μM, about 1.3 μM, about 1.4 μM, about 1.5 μM, about 1.6 μM, about 1.7 μM, about 1.8 μM, about 1.9 μM, about 2 μM, about 2.1 μM, about 2.2 μM, about 2.3 μM, about 2.4 μM, about 2.5 μM, about 2.6 μM, about 2.7 μM, about 2.8 μM, about 2.9 μM, about 3 μM, about 3.1 μM, about 3.2 μM, about 3.3 μM, about 3.4 μM, about 3.5 μM, about 3.6 μM, about 3.7 μM, about 3.8 μM, about 3.9 μM, about 4 μM, about 4.1 μM, about 4.2 μM, about 4.3 μM, about 4.4 μM, about 4.5 μM, about 4.6 μM, about 4.7 μM, about 4.8 μM, about 4.9 μM, about 5.0 μM.

Step b) of the method of the invention comprises contacting the target cell with a viral vector and the peptide according to step a).

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs that allow for random or site-directed integration of the targeting construct into genomic DNA. Such targeting constructs, preferably, comprise DNA of sufficient length for either homologous recombination or heterologous integration.

In a particular embodiment of the method of the invention, the vector is an expression vector. The term "expression vector" refers to a replicative DNA construct used for expressing the nucleic acid construct of the invention in a cell, preferably a eukaryotic cell, more preferably a mammalian cell. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

According to the invention, the vector used in step b) of the method of the invention is a viral vector or virus comprising the nucleic acid to be transduced into the target cell. In a preferred embodiment of the method of the invention the vector is a retroviral vector.

The term "retroviral vector" used herein means a replication deficient retroviral virus particle, which can transfer a foreign imported RNA of a gene or a fragment thereof or a reporter gene, e.g. a therapeutic gene, instead of the retroviral mRNA. The term "therapeutic gene" used herein refers to a nucleic acid sequence, which is introduced into a target cell by a retroviral vector and may comprise, without limitation, entire genes and fragments thereof, antisense nucleic acids and related sequences.

In a more particular embodiment, the retroviral vector is a lentiviral vector. The term "lentiviral vector", as used herein, refers to a vector based on a group (or scientific genus) of retroviruses that in nature give rise to slowly developing disease due to their ability to incorporate into a host genome. Modified lentiviral genomes are useful as viral vectors for the delivery of a nucleic acid sequence to a cell. An advantage of lentiviruses for infection of cells is the ability for sustained transgene expression. These viruses include in particular Human Immunodeficiency Virus type 1 (HIV-1), Human Immunodeficiency Virus type 2 (HIV-2), Simian Immunodeficiency Virus (SIV), Feline Immunodeficiency Virus (FIV), Equine Infectious Anaemia Virus (EIAV), Bovine Immunodeficiency Virus (BIV), Visna Virus of sheep (VISNA) and Caprine Arthritis-Encephalitis Virus (CAEV). Recombinant lentiviral vectors are capable of infecting non-dividing cells and can be used for both *in vitro, in vivo* and ex *vivo* gene transfer and expression of nucleic acid sequences. For example, recombinant lentivirus capable of infecting a non-dividing cell wherein a suitable host cell is transfected with two or more vectors carrying the packaging functions, namely gag, *pol* and *env,* as well as *rev* and *tat* is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific. The lentiviral vectors according to the invention may be genetically modified in such a way that certain genes constituting the native infectious virus are eliminated and replaced with a nucleic acid sequence of interest to be introduced into the target cells. The term "adenoviral vector", as used herein, refers to a vector based on an adenovirus. The term "adenovirus" refers to any virus pertaining to the Adenoviridae family characterized by being a non-enveloped virus with a pseudo-icosahedral nucleocapsid containing a double stranded DNA genome. This term includes any adenovirus capable of infecting a human or an animal, including all groups, subgroups, and serotypes that use CAR, CD46 or desmoglein-2 as receptor for infection of target cells. The term adenovirus includes, without limitation, avian, canine, equine, bovine, ovine, porcine, human or frog adenovirus. In a particular embodiment, the adenovirus is a human adenovirus, i.e. an adenovirus capable of infecting humans. A "serotype" is each of the immunologically different types of adenovirus. There are at least 57 serotypes of human adenovirus that are classified into several subgroups (A to G). In another embodiment, the viral vector is a vector based on a virus or the Parvoviridae family, preferably from the Parvovirinae subfamily, more preferably from the Dependoparvovirus genus, and yet even more preferably an adeno-associated virus.

In a preferred embodiment of the method of the invention, the retroviral vector is a pseudotyped lentiviral vector. The term "pseudotyped" and/or "pseudotyped vector" as used herein, means that the lentiviral vector comprises a virus core of a lentivirus and the virus envelope originating from a different lentivuris. In a preferred embodiment of the method of the invention, the lentiviral vector is pseudotyped with the G protein of the vesicular stomatitis virus (VSV-G).

The term "G protein of the vesicular stomatitis virus" or "VSV-G" refers to a type III viral fusion glycoprotein with a wide tropism, which binds to a low-density lipoprotein (LDL) receptor which is widely distributed across cells, allowing the membrane of the cell and the virus to fuse, and the contents of the virus interior to be inserted into the cell.

In a preferred embodiment of the method of the invention, the viral vector comprises a transgene under control of a promoter, in particular wherein the transgene encodes a therapeutic protein or a chimeric antigen receptor (CAR).

The term "transgene" means a heterologous gene derived from a source other than the wild type transgenic viral vector. The term transgene can also include a gene homolog derived from the wildtype of the transgenic viral vector or of the target cell.

The term "therapeutic protein" as used herein includes proteins, polypeptides, peptides, antibodies, and biologics (terms peptides, proteins, and polypeptides are used synonymously herein), which can be used as a treatment.

It is particularly contemplated that the term "therapeutic protein" includes antibodies, fragments, and variants thereof. Thus, the protein of the invention may comprise at least one fragment or variant of the therapeutic protein and / or at least one fragment or variant of the antibody.

The term "treatment" as used herein refers to an intervention (e.g., the administration of a therapeutic protein) which cures, ameliorates or lessens the symptoms of a disease or removes (or lessens the impact of) its cause(s) (for example, the eradication of cancer cells from the subject). In this case, the term is used synonymously with the term "therapy".

The term "chimeric antigen receptor" or "CAR" refers to engineered antigen receptors, which are grafted onto cells. In general, a CAR comprises an extracellular domain (extracellular part) comprising the antigen binding domain, a transmembrane domain and an intracellular signalling domain. The antigen-binding domain of the CAR targets specific antigens. The targeting regions may comprise full length heavy chain, Fab fragments, scFvs, divalent single chain antibodies or diabodies, each of which are specific to the target antigen. The antigen binding domain can be derived from the same species or a different species for or in which the CAR will be used in.

In a preferred embodiment of the method of the invention the viral vector is used at a MOI of between about 0.5 to about 25, preferably at a MOI of about 1, more preferably at a MOI of about 1, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25.

In a preferred embodiment of the method of the invention, step (b) is carried out after step (a) and for between about 0.5 days to about 20 days, preferably between about 1 and 15 days, more preferably between about 3 and 7 days, more preferably for 1, 2, 3, 4, 5, 6, 7, 8, 9, 10, 11, 12, 13, 14, 15, 16, 17, 18, 19, 20 days.

The method of the invention can further comprise other steps. In a preferred embodiment of the method of the invention the method further comprises contacting and incubating the target cell with a transducing enhancing compound selected from poloxamer 338, LAH4-A4, recombinant fibronectin-CH-296, prostaglandin E and protamine salt.

The method of the invention has the effect of increasing the level of transduction of a target cell when transduced with a viral vector, to values above the transduction of the same target cell with another method. Examples of how to determine the increase in transduction are described in the Examples section of the present description. In a preferred embodiment, the method of the invention improves the yield of transduction by at least 1%, at least 10%, at least 20%, at least 30%, at least 40%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, or more in comparison with a method of transduction which uses a conventional transducer improver such as Lentiboost®.

*Conjugate for enhancing transduction*

All previous definitions and embodiments described are equally valid and applicable to the present aspect and embodiments.

The discovery by the inventors that intermediate filaments can improve the transduction lead to the development of products with improved characteristics that could be used in a method, such as the method of the invention, for increasing the yield of the transduction of target cells.

As such, another aspect of the present invention relates to a conjugate for enhancing transduction efficiency, from here onwards the conjugate of the invention, comprising:

(a) a peptide comprising, in order going from the N-terminal end to the C-terminal end:

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I] and
(iii) a third region consisting of an amino acid sequence of "m" amino acids, wherein "m" is 0 to 41 amino acids,

wherein said peptide has a minimum length of 9 amino acids and a maximum length of 50 amino acids, and

(b) an amphiphilic polymer,

wherein the amphiphilic polymer is conjugated to the peptide.

In a preferred embodiment of the conjugate of the invention, the peptide has a minimum length of 12 amino acids and a maximum length of 20 amino acids.

In a preferred embodiment of the conjugate of the invention, the first region of the peptide comprises or consists of the sequence according to SEQ ID NO: 1.

In a preferred embodiment of the conjugate of the invention, the peptide comprises or consists of the sequence according to SEQ ID NO: 3.

In a preferred embodiment of the conjugate of the invention, the peptide comprises in the first region and/or the third region a cell penetrating peptide.

In a preferred embodiment of the conjugate of the invention, the peptide comprises three or more (i.e. 3, 4, 5, 6, 7, 8 or more) sequential Lysine amino acids (KKK), three or more (i.e. 3, 4, 5, 6, 7, 8, or more) sequential Arginine amino acids (RRR) or three or more (i.e. 3, 4, 5, 6, 7, 8, or more) sequential Histidine amino acids (HHH). In a more preferred embodiment, the cell penetrating peptide comprises eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

In another preferred embodiment of the conjugate of the invention, the peptide of the method of the invention comprises or consist of the sequence according to SEQ ID NO: 7.

In a preferred embodiment of the conjugate of the invention, the amphiphilic polymer is a poloxamer or a carboxylated derivative thereof, preferably the poloxamer F-127.

In a preferred embodiment of the conjugate of the invention, the molar ratio of peptide to amphiphilic polymers is of between 1:1 to 1:8, preferably between 1:2 to 1:6, more preferably of 1:4, more preferably the ratio is of 1:1, 1:2, 1:3, 1:4, 1:5, 1:6, 1:7, 1:8.

In a preferred embodiment of the conjugate of the invention, the peptide conjugated to the amphiphilic polymer forms particles (micelles).

In a preferred embodiment of the conjugate of the invention, the particles have a diameter of about 20 nm to about 30 nm.

The conjugate of the invention finds use in enhancing the transduction of a target cell. As such, another aspect of the present invention relates to the use of the conjugate of the invention for enhancing the transduction of a target cell.

_Method for obtaining the conjugate of the invention_

All previous definitions and embodiments described are equally valid and applicable to the present aspect and embodiments.

Another aspect of the present invention relates to a method for obtaining the conjugate of the invention. The aspect refers to a method for obtaining a conjugate according to the invention, from here onwards the method of conjugation of the invention, comprising:

(a) providing a peptide comprising, in order going from the N-terminal end to the C-terminal end:

(i) a first region consisting of an amino acid sequence of "n" amino acids, wherein "n" is 0 to 41 amino acids;

(ii) a second region consisting of the amino acid sequence [A/I/L/S/T/V]-[K/R]-[L/I/M/S/T/-V/A]-[G/R/A/H/K/S/F]-[L]-[D/E]-[IiNN IM/K/QI A/L/G/C]-[E]-[I] and

(iii) a third region consisting of an amino acid sequence of "m" amino acids, wherein "m" is 0 to 41 amino acids,

wherein said peptide has a minimum length of 9 amino acids and a maximum length of 50 amino acids, and an amphiphilic polymer, and

(b) conjugating the peptide with an activated form of the amphiphilic polymer or a derivative thereof wherein said activated form of the amphiphilic polymer contains a reactive group which is capable of reacting with at least one group in the peptide and wherein the contact is carried out under conditions adequate for the formation of a bond between the reactive group in the amphiphilic polymer and the group in the peptide.

The term "reactive group" as used herein refers to any group suitable for reaction with an amine group, preferably a carboxyl group, to bond the peptide to the amphiphilic polymer. In a preferred embodiment of the method of conjugation of the invention, the reactive group which reacts with at least one of the groups of the peptide is a carboxyl group.

In a preferred embodiment of the method of conjugation of the invention, the concentration of the amphiphilic polymer is of between about 15 mg/ml to about 45 mg/ml, preferably about 20 mg/mL to about 40 mg/mL.

A further aspect of the invention relates to a conjugate for enhancing transduction efficiency obtained by the method of conjugation of the invention.

The conjugate of the invention has the effect of increasing the level of transduction of a target cell when transduced with a viral vector, to values above the transduction of the same target cell with a method without the peptide according to the one described. Examples of how to determine the increase in transduction are described in the Examples section of the present description. In a preferred embodiment of the conjugate of the invention the conjugate improves the yield of transduction by at least 1%, at least 10%, at least 20%, at least 30%, at least 40%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, or more in comparison with a method of transduction which uses a conventional transducer improver such as Lentiboost®.

*Kit of the invention*

All previous definitions and embodiments described are equally valid and applicable to the present aspect and embodiments.

A further aspect of the present invention relates to a kit comprising a conjugate of the invention, from here onwards the kit of the invention.

In a preferred embodiment of the kit of the invention, the kit further comprises an additional transduction enhancing compound selected from poloxamer 338, LAH4-A4, recombinant fibronectin-CH-296, prostaglandin E and protamine salt.

In the context of the present invention, "kit" is understood as a product comprising the conjugate of the invention, and which may further comprise the different reagents for performing the methods described in the present invention. In such cases, where the kit further comprises different reagents, these are packaged together to allow for transport and storage. Nevertheless, if the kits defined in the present invention do not comprise the reagents necessary for putting the methods of the invention into practice, such reagents are commercially available and can be found as part of a kit. Suitable materials for packaging the components of the kit include, without being limited to, glass, plastic (polyethylene, polypropylene, polycarbonate and the like), bottles, vials, paper, sachets and the like. The kit of the invention can contain instructions for the sequential or separate use of the components in the kit. Said instructions can be in the form of printed material or in the form of an electronic support, capable of storing the instructions such that they can be read by a subject, such as electronic storage media (magnetic disks, tapes and similar), optical media (CD-ROM, DVD) and the like. Additionally or alternatively, the media can contain Internet addresses, such as in the form of a QR code, providing said instructions.

A further aspect of the invention relates to the use of the kit of the invention for the transduction of a target cell.

The present invention is further defined by the following aspects:

1. An *in vitro* method for transducing a target cell comprising:

(a) providing a peptide comprising, in order going from the N-terminal end to the C-terminal end

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/-F/V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids, wherein said peptide has a minimum length of 9 amino acids and a maximum length of 50 amino acids,

(b) contacting the target cell with a viral vector and the peptide according to step (a), and
(c) incubating the target cell in a culture medium.

2. The *in vitro* method according to aspect 1, wherein the peptide has a minimum length of 12 amino acids and a maximum length of 20 amino acids.

3. The *in vitro* method according to aspect 1 or 2, wherein the peptide comprises the sequence according to SEQ ID NO: 3.

4. The *in vitro* method according to any one of aspects 1 to 3, wherein the peptide further comprises in the first region and/or the third region a cell penetrating peptide.

5. The *in vitro* method according to aspect 4, wherein the cell penetrating peptide comprise a polar amino acid sequence, more preferably three or more sequential Lysine amino acids (KKK), three or more sequential Arginine amino acids (RRR) or three or more sequential Histidine amino acids (HHH); and most preferably eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

6. The *in vitro* method according to aspect 5 wherein the the peptide comprises the sequence according to SEQ ID NO: 8.

7. The *in vitro* method according to any one of aspects 1 to 6, wherein the peptide is conjugated to an amphiphilic polymer.

8. The *in vitro* method according to aspect 7, wherein the peptide conjugated to the amphiphilic polymer forms particles (micelles).

9. The *in vitro* method according to aspect 8, wherein the particles have a diameter of about 20 nm to 30 nm.

10. The *in vitro* method according to any one of aspects 7 to 9, wherein the molar ratio of peptide to amphiphilic polymers is of between 1:1 to 1:8, preferably between 1:2 to 1:6, more preferably of 1:4.

11. The *in vitro* method according to any one of aspects 7 to 10, wherein the amphiphilic polymer is selected from a group consisting of: poloxamer or a carboxylate derivative thereof, polyvinyl caprolactam-polyvinyl acetate-polyethylene glycol graft co-polymer, D-$\alpha$-Tocopherol polyethylene glycol 1000 succinate, poloxamines, poly(ethylene glycol)-block-poly($\epsilon$-caprolactone), and Poly(lactide-co-glycolide)-block-poly(ethylene glycol).

12. The *in vitro* method according to aspect 11 wherein the amphiphilic polymer is a poloxamer F-127.

13. The *in vitro* method according to any one of aspects 1 to 12, wherein in step (b) the peptide is used at a concentration of about 0.5 $\mu$M to about 5 $\mu$M, preferably of 1 $\mu$M to about 3 $\mu$M, more preferably about 1.5 $\mu$M.

14. The *in vitro* method according to any one of aspects 1 to 13, wherein the viral vector is a retroviral vector.

15. The *in vitro* method according to aspect 14, wherein the retroviral vector is a lentiviral vector, preferably pseudotyped with the G protein of the vesicular stomatitis virus (VSV-G).

16. The *in vitro* method according to any one of aspects 1 to 15, wherein the viral vector comprises a transgene under control of a promoter, in particular wherein the transgene encodes a therapeutic protein or a chimeric antigen receptor (CAR).

17. The *in vitro* method according to any one of aspects 1 to 16, wherein the viral vector is used at a multiplicity of infection (MOI) of between about 0.5 to about 25, preferably at a MOI of about 1.

18. The *in vitro* method according to any one of aspects 1 to 17, wherein the target cell is a mammalian cell or a murine cell, in particular a human cell.

19. The *in vitro* method according to aspect 18, wherein the target cell is a cell selected from the group consisting of a lymphocyte, a tumor cell, a lymphoid lineage cell, a neuronal cell, an epithelial cell, a keratinocyte, an endothelial cell, a primary cell, , a haematopoietic cell, and a stem cell.

20. The *in vitro* method according to aspect 19, wherein the haematopoietic cell is a haematopoietic stem cell, a haematopoietic progenitor cell, a CD34+ cell, a monocyte, a macrophage, a tissue resident macrophage, a microglial cell, or a dendritic cell.

21. The *in vitro* method according to aspect 18, wherein the murine cell is NIH/3T3, RAW64 or A-20.

22. The *in vitro* method according to aspect 18, wherein the human cell is HEK-293T.

23. The *in vitro* method according to aspect 19, wherein the lymphocyte cell is a T cell, a B cell or a natural killer cell.

24. The *in vitro* method according to any one of aspects 1 to 23, wherein the method further comprises contacting and incubating the target cell with a transducing enhancing compound selected from poloxamer 338, LAH4-A4, recombinant fibronectin-CH-296, prostaglandin E and protamine salt.

25. The *in vitro* method according to any one of aspects 1 to 24, wherein step (b) is carried out after step (a) and for between about 0.5 days to about 20 days, preferably between about 1 and 15 days, more preferably between about 3 and 7 days.

26. A conjugate for enhancing transduction efficiency comprising:

(a) a peptide comprising, in order going from the N-terminal end to the C-terminal end,

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids,

wherein said peptide has a minimum length of 9 amino acids and a maximum length of 50 amino acids, and
(b) an amphiphilic polymer,
wherein the amphiphilic polymer is conjugated to the peptide.

27. The conjugate according to aspect 26, wherein the peptide has a minimum length of 12 amino acids and a maximum length of 20 amino acids.

28. The conjugate according to aspect 26 or 27, wherein the peptide further comprises in the first region and/or the third region a cell penetrating peptide.

29. The conjugate according according to aspect 28, wherein the peptide comprises the sequence according to SEQ ID NO: 8.

30. The conjugate according to aspect 29 wherein the cell penetrating peptide comprise a polar amino acid sequence, more preferably three or more sequential Lysine amino acids (KKK), three or more sequential Arginine amino acids (RRR) or three or more sequential Histidine amino acids (HHH); and most preferably eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

31. The conjugate according to any one of aspects 26 to 30, wherein the conjugated is in the form of particles (micelles).

32. The conjugate according to aspect 31, wherein the particles have a diameter of about 20 nm to 30 nm.

33. The conjugate according to any one of aspects 26 to 32, wherein the molar ratio of peptide to amphiphilic polymer is of between 1:1 to 1:8, preferably between 1:2 to 1:6, more preferably of 1:4.

34. The conjugate according to any one of aspects 26 to 33, wherein the amphiphilic polymer is selected from a group consisting of: poloxamer or a carboxylate derivative thereof, polyvinyl caprolactam-polyvinyl acetate-polyethylene glycol graft co-polymer, D-$\alpha$-Tocopherol polyethylene glycol 1000 succinate, poloxamines, poly(ethylene glycol)-block-poly($\epsilon$-caprolactone), and Poly(lactide-co-glycolide)-block-poly(ethylene glycol).

35. The conjugated according to aspect 34 wherein the amphiphilic polymer is a poloxamer F-127.

36. The use of the conjugate according to any one of aspects 26 to 35 for enhancing the transduction of a target cell.

37. A method for obtaining a conjugate according to aspects 26 to 37 comprising:

(a) providing a peptide comprising, in order going from the N-terminal end to the C-terminal end,

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids,

wherein said peptide has a minimum length of 9 amino acids and a maximum length of 50 amino acids, and

an amphiphilic polymer, and

(b) conjugating the peptide with an activated form of the amphiphilic polymer or a derivative thereof wherein said activated form of the amphiphilic polymer contains a reactive group which is capable of reacting with at least one group in the peptide and wherein the contact is carried out under conditions adequate for the formation of a bond between the reactive group in the amphiphilic polymer and the group in the peptide.

38. The method according to aspect 37, wherein the peptide has a minimum length of 12 amino acids and a maximum length of 20 amino acids.

39. The method according according to aspect 37 or 38, wherein the peptide comprises the sequence according to SEQ ID NO: 3.

40. The method according to any one of aspects 37 to 39, wherein the peptide further comprises in the first region and/or the third region a cell penetrating peptide.

41. The method according to aspect 40 wherein the cell penetrating peptide comprise a polar amino acid sequence, more preferably three or more sequential Lysine amino acids (KKK), three or more sequential Arginine amino acids (RRR) or three or more sequential Histidine amino acids (HHH); and most preferably eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

42. The method according according to aspect 41, wherein the peptide comprises the sequence according to SEQ ID NO: 8.

43. The method according to any one of aspects 37 to 42, wherein the amphiphilic polymers is selected from a group consisting of: poloxamer or a carboxylate derivative thereof, polyvinyl caprolactam-polyvinyl acetate-polyethylene glycol graft co-polymer, D-$\alpha$-Tocopherol polyethylene glycol 1000 succinate, poloxamines, poly(ethylene glycol)-block-poly($\varepsilon$-caprolactone), and Poly(lactide-co-glycolide)-block-poly(ethylene glycol).

44. The method according to aspect 43 wherein the amphiphilic polymer is a poloxamer F-127.

45. The method according to any one of aspects 37 to 44 wherein the group which reacts with at least one of the groups of the peptide is a carboxyl group.

46. The method according to any one of aspects 37 to 45 wherein the concentration of the amphiphilic polymer is of between about 15 mg/ml to about 45 mg/ml, preferably about 20 mg/mL to about 40 mg/mL.

47. A conjugate for enhancing transduction efficiency obtained by the method according to any one of aspects 37 to 46.

48. A kit comprising a conjugate according to any one of aspects 26 to 36 or according to aspect 47.

49. The kit according to aspect 48 further comprising an additional transduction enhancing compound selected from poloxamer 338, LAH4-A4, recombinant fibronectin-CH-296, prostaglandin E and protamine salt.

50. Use of the kit according to aspect 48 or 49 for the transduction of a target cell.

The invention will be described by way of the following examples which are to be considered as merely illustrative and not limitative of the scope of the invention.

## EXAMPLES

### Example 1: METHODS

In order to facilitate their solubility, the peptides are covalently conjugated with polymeric micelles, based on the poloxamer F-127 (Pluronic™) and its carboxylated derivative (in a 4:1 ratio), that are used to bind to the peptides through their amino groups (amide bonds). Pluronics or poloxamers are non-ionic block copolymers of polypropylene oxide) (PPO) and poly(eythylene oxide) (PEO) (Batrakova EV et al. J Control Release. 2008; 130: 98).

The optimal concentration of the F-127 polymer was found to be in the range of 20 to 40 mg/mL, and the micellar peptides were adjusted to a final concentration of 500 μM, that was used as the stock concentration. This synthesis has been carried out by the Clinical Biochemistry, Drug Delivery and Therapy group (Drs. J. Seras, F. da Silva Andrade, D. Fernandes, VHIR) following the methodology described by Rafael *et al.,* (Doi: 10.1016/j.cej.2017.12.114).

All human samples were obtained after signed informed consent and the experiments using these samples were performed with the approval of our institutional Ethics Committee (CEIm).

### 1. Protocol for the use of micellar peptides:

1.1. Each stock of micellar peptide is at 500 μM (of peptide), and is dissolved in sterile milliQ water for storage. The micellar peptides were kept in a box at a constant temperature of 20° C, and were used by three months after conjugation.

1.2. To dilute the stocks for experimental use, we added phosphate buffer saline (PBS) at 10X to obtain an isotonic solution. The order of addition of the reagents is very important, if added in reverse order the micellar peptide may precipitate and loose activity.

1.3. From this isotonic solution, we added the necessary volume to obtain the desired final (working) concentration. We usually used in a range of concentrations between 1-3 μM since the peptides are cytotoxic at concentrations above 5 μM.

### 2. Lentiviral vectors

Three different lentiviral vectors (pCCL-PGK-eGFP, PGK-GFP-Wpre, and A3B1:CD8:4-1BB:CD3ζ) were used for these assays. The first two vectors, encoding an enhanced version of the green fluorescent protein (eGFP) under the constitutive PGK promoter, were kindly provided by Dr. Paula Rio (CIEMAT, Madrid, Spain), and the third one, encoding an anti-CD19 chimeric antigen receptor (CAR) was provided by the group of Dr. Sonia Guedán (IDIBAPS, Barcelona, Spain). All of them are third generation lentiviral vectors and are pseudotyped with the G protein of the vesicular stomatitis virus (VSV-G).

### 3. Transduction enhancers (TEs) used

For the studies of transduction enhancement, the following TE were used:
Peptides and controls: DIF-P-8R, K18, K18-8R, Sc-K18-8R, 8R and micelle alone (see Table 1):

*Table 1: Peptides used in the study*

| Name | Amino Acid sequence | MW (Da) |
|------|---------------------|---------|
| DIF-P-8R | LLNVKMALDIEIRRRRRRRR (SEQ ID NO: 9) | 2621,2 |
| K18 | LLNIKVKLEAEIATYRRLLE (SEQ ID NO: 3) | 2385,9 |
| K18-8R | LLNIKVKLEAEIRRRRRRRR (SEQ ID NO: 8) | 2632,2 |
| Sc-K18-8R | LLNKEIKIEVALARRRRRRRR (SEQ ID NO: 10) | 2632,2 |
| 8R | RRRRRRRR (SEQ ID NO: 6) | 1393,6 |

DIF-P corresponds to the 12 AA natural sequence of the IF peripherin and vimentin containing the 9AA core sequence (VKMALDIEI - SEQ ID NO: 11), plus a tail of 8 arginine residues (8R - SEQ ID NO: 6) in its carboxy terminal end. K18 consists of a 20 AA natural sequence of the IF keratin 18 (K18) containing the 9 amino acids core sequence (IKVKLEAEI - SEQ ID NO: 1), K18-8R contains the first 12 amino acids of K18 plus an 8R tail, and Sc-K18-8R has the same amino acid composition as K18-8R, but with a scrambled sequence between amino 4 and amino 12 (the core sequence). In all cases, the peptides are conjugated to the polymeric micelles, which were used at 20 mg/mL, except for the transduction of the NK cells, which were used at 40 mg/ml.

The commercial TEs were used at the concentrations recommended by the manufacturers (Table 2):

*Table 2: Commercial TEs used*

| **Name** | **Manufacturer** | **Concentration used** |
|----------|------------------|------------------------|
| LentiBoost™ (LB) | Syrion Biotech | 1-5 mg/mL |

(continued)

| Name | Manufacturer | Concentration used |
|---|---|---|
| Vectofusin-1™ (VF-1) | Miltenyi Biotec | 10 μg/mL |
| Protamine sulfate (PS) | Sigma Aldrich | 4 μg/mL |
| Prostaglandin $E_2$ (PGE$_2$) (Dinoprostone™) | Leo Pharma | 10 μg/mL |
| Retronectin™ (RN) (coated surface) | Takara Bio | 48 μg/mL |

**4. Transduction protocol for cell lines.**

To carry out the transduction enhancement assays, 3000 cells (HEK-293T or NIH/3T3, both purchased at the ATCC) were seeded in 100 μL of culture medium (consisting of *Dulbecco's Modified Eagle Medium* (high glucose) (DMEM, Gibco™), supplemented with 10% fetal bovine serum (FBS), 1% L-glutamine and 1% penicillin/streptomycin (P/S) (both from Cultek S.L.U.), per well, in culture-treated flat-bottomed 96-well plates, and incubated for 24 h at 37° with 5% $CO_2$ and 100% relative humidity.

The vector was diluted in complete medium, and the appropriate amounts were added to each well at the chosen MOI into a final volume of 200 μL per well, in the absence or presence of the chosen TE (peptides, LB, VF-1, PS, RN, PGE$_2$) at different concentrations and following the manufacturer's recommendations. The culture medium was changed 24 h after transduction and the cells were incubated for 48 h more until the plate was analyzed 3 days post-transduction by flow cytometry.

All experiments included a non-transduced control (cells only) without vector exposure, and a control with vectors but without TE (basal transduction). In all assays, a minimum of three replicates (technical replicates) were performed per experimental condition, and were repeated a minimum of three times (experimental replicates) for statistical analysis.

**5. Transduction protocol for CD34$^+$ cells:**

1. The CD34$^+$ cells were isolated from apheresis products from healthy donors and cryopreserved in liquid nitrogen until use. Cells were then thawed, counted with trypan blue, and seeded in culture-treated 96-well flat-bottom plates, about 50.000 cells per well in 100 μL of complete StemSpan™ SFEM Hematopoietic Cell Culture Medium (SFEM medium) (Stem Cell Technologies™, Vancouver, Canada), and incubated at 37°C, 5% $CO_2$ and 100% relative humidity. The complete medium was supplemented with recombinant human stem cell factor (rhSCF), recombinant human flt3 ligand (rhFlt3-L) and recombinant human thrombopoietin (rhTpo) (all from EuroBioScience, Friesoythe, Germany).

2. After 24 h in culture, the cells were transduced as follow: Previously, the required volume of lentiviral vector was calculated in accordance with the vector titer, the CD34$^+$ target cell number and the desired MOI. Prior to its use, the vector was diluted in complete SFEM medium.

3. Next, all TEs used in the assay (peptides, LB, VF-1, PS, RN, PGE$_2$) were prepared at the desired concentrations following the corresponding manufacturer's recommendations. Then, the TEs and the lentiviral vectors were added to a final volume of 200 μL per well, and the plates were incubated at 37°C, 5% $CO_2$, and 100% relative humidity.

4. After 24 h post-transduction, 90% of the supernatant (containing the lentiviral vector) was removed from the cell culture, and complete fresh medium was added again and incubated at 37°C, 5% $CO_2$, and 100% relative humidity.

5. Finally, at 3, 7 and 14 days post-transduction, the cells were analyzed by flow cytometry and the plates were stored at -80° for future analyses.

**6. Transduction protocol for human (CD4$^+$) T cells:**

1. After isolation and activation of the CD4$^+$ T cells, a suspension of $10^6$ cells/mL was prepared and 100 μL were added per well to a 96-well flat-bottom plate and incubated at 37°C, 5% $CO_2$, and 100% relative humidity. After 24 h in culture, the vector was diluted to the desired concentration and mixed with the different TEs (peptides, LB, VF-1, PS, $PGE_2$), and 50 μL per well of the vector-TEs solution was added. The A3B1:CD8:4-1BB:CD3ζ (also named ARI001-CodonOptimized3 vector), a third generation lentiviral vector encoding an anti-human CD19 CAR, was used for this assay.

2. After 72 h post-transduction, samples were collected and stained for flow cytometry analysis: First, the entire plate contents were transferred to a pre-labeled 96-well roundbottom plate. Two washes were performed with 1X PBS, adding 100 μL per well and centrifuging the plate for 2' at 2000 rpm. In the last wash, the supernatant was discarded, and 100 μL of a working solution of eFluor™ 450 - Fixable Viability Dye Live/Dead (eBioscience™) was added and incubated for 30' at RT.

3. Two washes were performed with 200 μL of FACS buffer (PBS 1X + 3% FBS). After the last wash, the supernatant was removed and 100 μL of FACS buffer containing 2 μL of biotinylated goat anti-mouse or anti-human (Fab)2 was added for CAR protein staining. The cells were resuspended and incubated at 4° for 30'.

4. After two washes were performed with FACS buffer, adding 100 μL per well and centrifuging the plate for 2' at 2000 rpm. After the last wash, the supernatant was discarded, and 100 μL of FACS buffer containing 1 μL of PE-streptavidin was added, mixed well and incubated at 4° for 30'.

5. Then, two additional washes were performed with 200 μL of FACS buffer. After the last wash, the supernatant was removed and 200 μL of freshly prepared 2% paraformaldehyde in 1X PBS was added. Finally, transduction efficiency was analyzed by flow cytometry to determine the percentage of CAR-positive T cells.

This protocol was provided by Drs. Sonia Guedán and Marta Giménez (IDIBAPS, Hospital Clinic, Barcelona), and these experiments were performed at their laboratory.

**7. Transduction protocol for human NK cells:**

1. Total blood samples were collected and processed in less than 4 h. After obtaining the peripheral blood mononuclear cells (PBMCs) by density gradient centrifugation (Ficoll), NK cells (following the instructions of the Miltenyi Biotec isolation kit) from human they were seeded in a 24-well plate [$10^6$ cells per well in 1 mL of complete medium (NK MACS supplemented with 5% AB serum (Tebu-Bio, Le Perray, France), recombinant human IL-2 (rhIL-2) and recombinant human IL-15 (rhIL-15), both from Miltenyi Biotec)], and incubated at 37°C, 5% $CO_2$, and 100% relative humidity.

2. After 48 h in culture, cells were counted, and about 40,000 cells were seeded in 100 μL of complete medium (per well) in culture-treated 96-well flat-bottom plate. The transduction process was performed as follows:

3. Calculate the required volume of lentiviral vector for a known number of NK cells and desired MOI, and the vector diluted in complete medium. Simultaneously, prepare all the enhancers to be used in the assay (micellar peptides, LB, PS, VF-1, etc.) at the concentrations recommended by the manufacturers. For these experiments, we used the peptides at 1.5 μM, LB at 5 mg/mL, PS at 4 μg/mL and VF-1 at 2.5 μg/mL.

4. First, we added the micellar peptides and TEs to the culture and the lentiviral vector, into a final volume of 200 μL per well, and incubated the cells at 37°C, 5% $CO_2$ and 100% relative humidity.

5. After 24 h post-transduction, the supernatant of the NK cell culture is carefully removed, fresh complete medium was added, and the cells were incubated at 37°C, 5% $CO_2$, and 100% relative humidity.

6. At days 3, 7 and 14 post-transduction, the cells were analyzed by flow cytometry.

**8. Flow Cytometry**

Transduction efficiency was analyzed using an LSR Fortessa cell analyzer (Becton, Dickinson, New Jersey, USA), after 3, 7 or 14 days post-transduction (depending on the target cell type). First, cells were transferred using a multichannel pipette to a U-bottom 96-well plate. Live and dead cells were dis-criminated using 1 μg/mL 7-amino-

actinomycin D (7-AAD).

To analyze the transduction efficacy and cytotoxicity of the compounds used, different gates of analysis were defined. Samples of transduced cells (labeled and unlabeled with PI or 7-AAD), with a minimum of 10.000 events in the relevant gate, were acquired. Further, appropriate thresholds were established to define the cell populations. Four final gates were created and applied sequentially:

- Gate 1. Plotting forward scatter high or FSC-H (Forward scatter height) versus forward scatter area or FSC-A (Forward scatter area). Here single events were gated to eliminate doublets.

- Gate 2. Plotting the Side scatter area (SSC-A) against the Forward scatter area (FSC-A). This gate was used to eliminate cellular debris and atypical and undesired events, taking into account their morphology and complexity.

- Gate 3. Thanks to the labeling of the cells with PI or 7-AAD it was possible to calculate the percentage of dead cells and eliminate them from the study. Only live cells were considered for the subsequent analysis gate.

- Gate 4. The percentage of live transduced cells was obtained using eGFP fluorescence (FL1). As an additional parameter, the Mean Fluorescence Intensity (MFI) of the transduced cells was measured. The MFI is the mean of all fluorescence intensities of individual cells (or events) in a desired population. In our case, the MFI values obtained represented the mean fluorescence level of eGFP in the transduced cells (gate 4). All data were obtained and analyzed using FACSDiva software v6.2 and FlowJo v10 software (both from BD Biosciences).

### 9. Vector copy number (VCN)

The average number of vector genome copies per cell was also assessed by qPCR (Rio P et al. Nat Med 2019) in the transduced CD34$^+$ cells. Briefly, after genomic DNA isolation from the transduced cells, quantitative PCR (qPCR) was used to determine VCN. The gDNA was amplified by qPCR with primers and specific fluorescent probes (Table 1), using the ABsolute qPCR Low ROX Mix master mix (ThermoFisher Scientific) shown in Table 2, on a QuantStudio™ system. 5 Flex Real-Time PCR (UAT).

After the previous steps of 50°C for 2' and 95°C for 10', 40 consecutive cycles of 95°C/15" and 60°C/1' were performed for gDNA amplification (Table 3 and 4).

Table 3. Primers and probes used to determine VCN

|  |  | Sequence (5'-3') | Tm (°C) |
|---|---|---|---|
| **Primers** | Alb. F | GCTGTCATCTCTTGTGGGCTG (SEQ ID NO: 12) | 64 |
|  | Alb. R | ACTCATGGGAGCTGCTGGTTC (SEQ ID NO: 13) | 63 |
|  | Ψ. F | CAGGACTCGGCTTGCTGAAG (SEQ ID NO: 14) | 63 |
|  | Ψ. R | TCCCCCGCTTAATACTGACG (SEQ ID NO: 15) | 59 |
| **Probes** | Alb. P | [VIC]-CCTGTCATGCCCACACAAATCTCTCC-[BHQ1] (SEQ ID NO: 16) | 73.9 |
|  | Ψ. P | [FAM]-CGCACGGCAAGAGGCGAGG-[BHQ1] (SEQ ID NO: 17) | 75.7 |

Abbreviations: Alb (Albumin), BHQ-1 (black hole quencher-1: molecule that absorbs and suppresses the fluorescent signal), F (Forward), R (Reverse), Ψ (Psi, encapsidation sequence), FAM and VIC (probes multiplexed fluorescent hydrolysis).

Table 4. Mixture used in the qPCR. Abbreviations: Alb (Albumin), F (Forward), R (Reverse), gDNA (genomic DNA), Ψ (Psi, encapsidation signal).

|  | **Concentration stock** | **Concentration Mix** | **Volume PCR Mix** |
|---|---|---|---|
| **Absolute** | 2X | 1X | 12.5 μL |
| **Alb. F** | 10 μM | 0.10 μM | 0.25 μL |
| **Alb. R** | 10 μM | 0.10 μM | 0.25 μL |
| **Alb. P** | 10 μM | 0.10 μM | 0.25 μL |
| **Ψ. F** | 10 μM | 0.10 μM | 0.25 μL |

(continued)

|  | Concentration stock | Concentration Mix | Volume PCR Mix |
|---|---|---|---|
| $\Psi$. R | 10 $\mu$M | 0.10 $\mu$M | 0.25 $\mu$L |
| $\Psi$. P | 10 $\mu$M | 0.10 $\mu$M | 0.25 $\mu$L |
| gDNA (10 ng/$\mu$L) | - | 100 ng | 10 $\mu$L |
| H$_2$O | - | - | 1 $\mu$L |
| V$_{final}$ of reaction |  |  | 25 $\mu$L/well |

The VCN measures the number of integrated copies of the LV vector per transduced cell. To do this, the proviral genome, specifically a region of the Psi packaging sequence, was amplified together with the host genome, using a sequence of the albumin (Alb) gene as a reference. Quantitative amplification by qPCR provided the cycle threshold (Ct) value, which was interpolated from each sample into a standard curve with 10-fold dilutions of the plasmid comprising both Psi/Alb target sequences (pRRL.PGK.eGFP /ALB) to determine the number of copies of each sequence. The standard curve allowed the cell number to be calculated assuming that, as a diploid organism, the cells contain 2 copies of Alb. With all this data, the VCN was determined as follows:

$$\text{VCN (proviral copies/cell)} = \frac{\text{number of proviral copies } (Psi)}{(\frac{\text{number of Alb copies}}{2})}$$

This protocol was provided by Dr. Paula Rio (CIEMAT, Madrid).

**10. Statistical analyses**

All tests were carried out in at least three independent experiments. Results are expressed as mean $\pm$ SD (standard deviation). Statistical analysis was performed with GraphPad Prism 9 software (Dotmatics, Boston, USA). Differences were considered statistically significant when the p value was less than 0.05 (*$p<0.05$, **$p<0.01$, ***$p<0.001$ and ****$p <0.0001$).

**RESULTS**

The results are shown in five figures corresponding to representative transduction experiments using a murine fibroblastic cell line (NIH/3T3) and a human fibroblastic cell line (HEK-293T) (figures 1 and 2, respectively) and the three main target primary cell types for ex *vivo* GT: Hematopoietic progenitors (purified CD34[+] cells) (figures 3 and 4), T-cells (CD4[+]) (figure 5), and NK cells (figure 6).

**Transduction enhancement**

NIH/3T3 cells

The experimental results showed that, in this cell line and under these specific experimental conditions, the highest transduction enhancing activity was observed with the micellar K18-8R peptide at 1 $\mu$M (Figure 1). The toxicities observed were similar in all the experimental conditions.

HEK-293T cells

To determine whether other peptides derived from other members of the IF family also had transduction enhancing ability, we performed a transduction experiment that included DIF-P-8R, which derives from the IF peripherin (or vimentin), in addition to the K18 peptides. Although the experiment was made only once, we used several concentrations of the peptides and two different MOI (1 and 5), and we observed that, in both cases, the highest transduction enhancement was observed with the K18-8R peptide at 1 $\mu$M). At higher concentrations (5 $\mu$M or more), this activity decreased, perhaps due to toxicity. DIF-P-8R also had enhancing activity, that was higher at 5-15 $\mu$M than at 1 $\mu$M. The micelle alone also showed a significant activity, that was dose-dependent, which is consistent with the effect of another TE based on a similar poloxamer, the LentiBoost™ (Figure 2).

In other experiments, comparing the transduction enhancing activity of different peptides derived from K18 and other commercial TE, the highest activity was observed with both K18 and K18-8R (Figure 3).

Human CD34$^+$ (hematopoietic) cells

Human CD34$^+$ cells were isolated from apheresis products obtained from healthy donors. The transduction efficiencies analyzed by flow cytometry, of a representative experiment, are shown below in Figure 4. Again, micellar K18-8R showed the highest enhancing efficacy, that was more evident at days 7 and 14 after transduction. Interestingly, the micellar scrambled peptide (Sc-K18-8R), the micelle alone and the LentiBoost™ also showed a significant enhancing activity, although the differences with respect to the basal transduction were statistically significant only for the K18-8R and the LentiBoost™. Molecular analysis (VCN) of the transduced samples confirmed these results (Figure 5).

Human (CD4$^+$) T cells

Human CD4$^+$ T cells were isolated from peripheral blood samples obtained from healthy donors. The transduction efficiencies were analyzed by flow cytometry using a labeled anti-CAR antibody, 72 h after transduction. Figure 6 represents the results of a representative experiment. LentiBoost, the two peptides (K18-8R and its scrambled control), and the micelle alone yielded similar results, with fold changes between 4 and 8. K18-8R resulted in the highest transduction efficiency, although the differences between these four compounds were not statistically significant.

Human NK cells

Human NK cells were isolated from peripheral blood samples obtained from normal donors, and were processed in fresh (in less than 4 h). Figure 7 represents the results of the transduction efficiencies analyzed by flow cytometry at different timepoints, corresponding to a representative experiment. In this experiment we also tested additional concentrations of the peptides (3 $\mu$M) and LB (5 mg/mL), but only the best results obtained are included in the charts (peptides at 1.5 $\mu$M) and LB at 2.5 mg/mL.

Again, the highest enhancing activity was observed with the micellar K18-8R peptide, with fold changes between 2.2 (day 3) and 3.5 (day 14), with a clear difference in comparison to the scrambled control and the micelle alone, and also superior to LB (although the differences between K18-8R and LB were not statistically significant).

**Mechanistic studies**

Additional studies have been carried out with these peptides on cytotoxic, hemolytic, antimicrobial activity, and assays to assess membrane permeability, such as Ca$^{2+}$ ion efflux and Yo-Pro entry (a high molecular weight fluorochrome that can only cross disrupted membranes), and activation of the canonical NLRP3 inflammasome with quantification of IL-1$\beta$ and IL-18 secretion upon exposure to the peptides and in the presence or absence of specific inhibitors of this inflammasome. Studies with the fluorochrome Yo-Pro-1 were performed at the laboratory of Dr. Pablo Pelegrin, at the University of Murcia. In summary, the results of these studies show that the K18-8R peptide increases membrane permeability in a dose-dependent manner (as long as the concentrations do not exceed 10-15 $\mu$M). Using higher concentrations, cytotoxic, hemolytic and antimicrobial activity predominated, also in a dose-dependent manner.

Additionally, we performed confocal microscopy studies using peptides labeled with a fluorochrome (Quasar 670). The results of these experiments showed that the peptide was localized to the cytoplasmic membranes, induced the production of blebs and eventually (at concentrations between 15 and 50 $\mu$M), cell death.

Taken together, these results are very suggestive of a mechanism of action mediated by the formation of pores or other structures in the membrane which, at high concentrations (50 $\mu$M), would produce cell death by apoptosis and pyroptosis, and at lower concentrations (5-15 $\mu$M) would activate the NLRP3 inflammasome, inducing the production of pro-inflammatory cytokines (specific inhibitors reduce the production of IL1$\beta$), and we also believe that it participates in the transduction enhancing effect.

**Claims**

**1.** An *in vitro* method for transducing a target cell comprising:

(a) providing a peptide comprising, in order going from the N-terminal end to the C-terminal end

(i) a first region consisting of an amino acid sequence of "n" amino acids, wherein "n" is 0 to 41 amino acids;

(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids,

wherein said peptide has a minimum length of 9 amino acids and a maximum length of 50 amino acids,
(b) contacting the target cell with a viral vector and the peptide according to step (a), and
(c) incubating the target cell in a culture medium.

2. The *in vitro* method according to claim 1, wherein the peptide comprises the sequence according to SEQ ID NO: 3.

3. The *in vitro* method according to claim 1 or 2, wherein the peptide further comprises in the first region and/or the third region a cell penetrating peptide.

4. The *in vitro* method according to claim 3, wherein the the peptide comprises the sequence according to SEQ ID NO: 8.

5. The *in vitro* method according to any one of claims 1 to 4, wherein the peptide is conjugated to an amphiphilic polymer.

6. The *in vitro* method according to claim 5 wherein the amphiphilic polymer is a poloxamer F-127.

7. The in vitro method according to any one of claims 1 to 6, wherein the viral vector is a retroviral vector.

8. A conjugate for enhancing transduction efficiency comprising:

(a) a peptide comprising, in order going from the N-terminal end to the C-terminal end,

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids,

wherein said peptide has a minimum length of 9 amino acids and a maximum length of 50 amino acids, and
(b) an amphiphilic polymer,

wherein the amphiphilic polymer is conjugated to the peptide.

9. The conjugate according to claim 8, wherein the peptide further comprises in the first region and/or the third region a cell penetrating peptide.

10. The conjugate according to claim 9, wherein the peptide comprises the sequence according to SEQ ID NO: 8.

11. The conjugate according to claim 10 wherein the cell penetrating peptide comprises a polar amino acid sequence, more preferably three or more sequential Lysine amino acids (KKK), three or more sequential Arginine amino acids (RRR) or three or more sequential Histidine amino acids (HHH); and most preferably eight or more sequential Lysine amino acids (KKKKKKKK, SEQ ID NO: 5), eight or more sequential Arginine amino acids (RRRRRRRR, SEQ ID NO: 6) or six or more sequential Histidine amino acids (HHHHHH, SEQ ID NO: 7).

12. The conjugated according to claim any one of claims 8 to 11, wherein the amphiphilic polymer is a poloxamer F-127.

13. Use of the conjugate according to any one of claims 8 to 12 for enhancing the transduction of a target cell.

14. A method for obtaining a conjugate according to any one of claims 8 to 13 comprising:

(a) providing a peptide comprising, in order going from the N-terminal end to the C-terminal end,

(i) a first region consisting of an amino acid sequence of "n" amino acids, wherein "n" is 0 to 41 amino acids;
(ii) a second region consisting of the amino acid sequence [A/I/L/S/T/V]-[K/R]-[L/I/M/S/T/-V/A]-[G/R/A/H/K/S/F]-[L]-[D/E]-[I/N/V/M/K/Q/A/L/G/C]-[E]-[I],
(iii) a third region consisting of an amino acid sequence of "m" amino acids, wherein "m" is 0 to 41 amino acids,

wherein said peptide has a minimum length of 9 amino acids and a maximum length of 50 amino acids, and an amphiphilic polymer, and

(b) conjugating the peptide with an activated form of the amphiphilic polymer or a derivative thereof, wherein said activated form of the amphiphilic polymer contains a reactive group which is capable of reacting with at least one group in the peptide and wherein the contact is carried out under conditions adequate for the formation of a bond between the reactive group in the amphiphilic polymer and the group in the peptide.

15. A conjugate for enhancing transduction efficiency obtained by the method according to claim 14.

Fig. 1

Fig. 2

Fig. 3

Fig. 4

Fig. 5

Fig. 6

Fig. 7

**EUROPEAN SEARCH REPORT**

Application Number

EP 24 38 2581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/260603 A1 (UNIV LLEIDA [ES] ET AL.) 30 December 2020 (2020-12-30) * see opinion for details * ----- | 1-4,7 | INV. A61K47/00 A61K49/00 C07K19/00 |
| Y | BODRATTI ANDREW ET AL: "Formulation of Poloxamers for Drug Delivery", JOURNAL OF FUNCTIONAL BIOMATERIALS, vol. 9, no. 1, 18 January 2018 (2018-01-18), page 11, XP055782626, DOI: 10.3390/jfb9010011 Retrieved from the Internet: URL:https://www.ncbi.nlm.nih.gov/pmc/articles/PMC5872097/pdf/jfb-09-00011.pdf> * see opinion for details * ----- | 1-15 | C07K17/00 A61K38/00 C12N15/86 |
| Y | JULIEN BALZEAU ET AL: "The vimentin-tubulin binding site peptide (Vim-TBS.58-81) crosses the plasma membrane and enters the nuclei of human glioma cells", INTERNATIONAL JOURNAL OF PHARMACEUTICS, ELSEVIER, AMSTERDAM, NL, vol. 423, no. 1, 28 April 2011 (2011-04-28), pages 77-83, XP028439789, ISSN: 0378-5173, DOI: 10.1016/J.IJPHARM.2011.04.067 [retrieved on 2011-05-07] * the whole document * ----- | 1-15 | |

TECHNICAL FIELDS
SEARCHED (IPC)

A61K
C07K
C12N

The present search report has been drawn up for all claims

| Place of search | Date of completion of the search | Examiner |
|---|---|---|
| Munich | 30 October 2024 | Landré, Julien |

EPO FORM 1503 03.82 (P4C01)

## EP 4 656 207 A1

EP 24 38 2581

30-10-2024

| Patent document cited in search report | Publication date | Patent family member(s) | | Publication date |
|---|---|---|---|---|
| WO 2020260603 A1 | 30-12-2020 | EP | 3756680 A1 | 30-12-2020 |
| | | EP | 3990002 A1 | 04-05-2022 |
| | | JP | 2022545765 A | 31-10-2022 |
| | | US | 2022251158 A1 | 11-08-2022 |
| | | WO | 2020260603 A1 | 30-12-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3756680 A1
- US 5994136 A

**Non-patent literature cited in the description**

- **FARMER, P. S**. Dmg Design. Academic Press, 1980, vol. 10, 119-143
- **BALL, J. B.** ; **ALEWOOD, P. F.** *J. Mol. Recognition*, 1990, vol. 3, 55
- **MORGAN, B. A.** ; **GAINOR, J. A.** *Ann. Rep. Med. Chem.*, 1989, vol. 24, 243
- **FREIDINGER, R. M.** *Trends Pharmacol. Sci.*, 1989, vol. 10, 270
- Peptidomimetic Design and Chemical Approaches to Peptide Metabolism. **SAWYER, T. K.** Peptide-Based Drug Design: Controlling Transport and Metabolism,. 1995
- **SMITH, A. B. 3 et al.** *J. Am. Chem. Soc.*, 1995, vol. 117, 11113-11123
- **SMITH, A. B. 3 et al.** *J. Am. Chem. Soc.*, 1994, vol. 116, 9947-9962
- **HIRSCHMAN, R. et al.** *J. Am. Chem. Soc.*, 1993, vol. 115, 12550-12568
- **JAMES, G. L. et al.** *Science*, 1993, vol. 260, 1937-1942
- **PASUT, G.** *Polymers*, 2014, vol. 6 (1), 160-178
- **SINGH,A** ; **KUMARI, K.** ; **PABAN, P.** *Physical Sciences Reviews*, 2023, 1-18
- **BATRAKOVA EV et al.** *J Control Release*, 2008, vol. 130, 98
- **DRS. J. SERAS** ; **F. DA SILVA ANDRADE** ; **D. FERNANDES, VHIR**. *Clinical Biochemistry, Drug Delivery and Therapy group*
- **RIO P et al.** *Nat Med*, 2019